Europäisches Patentamt

**European Patent Office**

**Office européen des brevets**

(19)

(11) Numéro de publication: **0 223 661**
A1

(12) ## DEMANDE DE BREVET EUROPEEN

(21) Numéro de dépôt: **86402304.9**

(22) Date de dépôt: **15.10.86**

(51) Int. Cl.⁴: **C 05 F 11/08, C 12 N 1/04**

(30) Priorité: **17.10.85 FR 8515396**

(43) Date de publication de la demande: **27.05.87**
**Bulletin 87/22**

(84) Etats contractants désignés: **AT BE CH DE ES FR GB GR IT LI LU NL SE**

(71) Demandeur: **SOCIETE FRANCAISE DE FERTILISATION " FERTIL FRANCE" AGRO BIO-INDUSTRIE, Site du Paradis, F-54980 Batilly (FR)**

(72) Inventeur: **Rambier, Guy Henri Marie, Domaine des Légers, F-18000 Sainte-Montaine (FR)**

(74) Mandataire: **Warcoin, Jacques et al, Cabinet Régimbeau 26, avenue Kléber, F-75116 Paris (FR)**

(54) **Inoculum microbien et procédé de préparation d'une poudre stable de bacteries, agent fertilisant et son application, procédé pour l'amélioration des sols.**

(57) La présente invention concerne un inoculum microbien, utile notamment pour la dégradation des matières organiques dans les sols, composé d'un mélange de microorganismes de genres différents pouvant vivre dans le sol et non-pathogènes pour l'homme, les animaux et les végétaux, caractérisé en ce qu'il s'agit de microorganismes aérobies stricts ou aérobies facultatifs, et mésophiles, existant sous une forme résistante au stockage, dégradant la cellulose, l'hémi-cellulose, la lignine et les protéines composant les matières organiques d'origine végétale ou animale.

EP 0 223 661 A1

ACTORUM AG

INOCULUM MICROBIEN ET PROCEDE DE PREPARATION D'UNE
POUDRE STABLE DE BACTERIES, AGENT FERTILISANT ET
SON APPLICATION, PROCEDE POUR L'AMELIORATION DES SOLS

Les rendements agricoles d'un sol, envisagés des points de vue qualitatif et quantitatif, sont bien sûr dépendants des facteurs climatiques, mais sont surtout directement proportionnels aux constitutions physique et chimique du sol.

Du point de vue chimique, l'eau, les substances minérales et les substances organiques constituent les facteurs essentiels de la richesse du sol. A cet égard, l'azote provenant de la dégradation des litières est particulièrement important mais est souvent perdu pour le sol, par formation d'ammoniac ($NH_3$). En effet, les litières, source principale de substances organiques, composées de débris végétaux et de cadavres d'animaux ou de bactéries, se décomposent à la surface du sol de deux façons :

La première voie de décomposition est une minéralisation rapide : 70 à 90 % de la matière organique est prise en charge par les microorganismes qui transforment en particulier l'azote en ammoniac, dont une partie se minéralise en nitrate ($NO_3^-$).

La seconde voie conduit à la formation d'humus, par formation lente de complexes colloïdaux organiques dont l'intérêt est de mettre en réserve de l'azote sous une forme non minéralisée.

Cet humus est par la suite lentement minéralisé (1 à 2 % par an).

Le problème de cette décomposition des litières en surface est qu'elle aboutit à une perte considérable d'azote sous forme de $NH_3$, de carbone sous forme de $CO_2$ et d'autres substances contribuant ainsi à un non-enrichissement du sol.

Il existe bien, dans les sols hydromorphes, une voie de dégradation anaérobie conduisant à la production de tourbe, mais cette minéralisation est lente et 30 % des matières organiques échappent à l'humidification, ce qui donne au sol une structure fibreuse néfaste.

C'est la volonté de récupérer toute cette énergie perdue pour le sol qui a conduit aux recherches qui sont à la base de la présente invention.

Les études concernant l'influence des microorganismes sur les systèmes de nutrition des végétaux ont, pour l'instant, essentiellement porté sur le phénomène de vie symbiotique ; mais même les microorganismes non symbiotiques étudiés n'ont pu être utilisés en agriculture que de façon très spécifique. Or, les cultures intensives (grande culture, céréales oléagineux...) exportent une grande quantité d'humus, qui souvent n'est pas restituée par l'agriculteur. Il s'en suit alors une baisse continue du taux de matière organique totale du sol et donc un risque certain de battance. Cette battance responsable d'un manque d'aération du sol a pour conséquence une mauvaise dégradation de la matière organique fraîche (résidu de récolte, paille, chaume, etc.) et par suite d'une augmentation de la flore fongique pathogène et donc d'une recrudescence des affections cryptogamiques pour les espèces végétales en culture.

D'autre part, il a été prouvé que la monoculture (céréales ou betterave par exemple) ou des rotations de cultures répétées (assolement) provoquaient des modifications au sein de la microflore du sol, en particulier la flore cellulolytique.

En effet, de nombreuses isolations et identifications à partir de prélèvements de sols cultivés ont montré une diminution de près de 50 à 75 % des bactéries cellulolytiques responsables en partie de la dégradation des résidus de récoltes (paille, chaume, etc.).

De plus, les cycles biologiques ou cycles d'assimilation des éléments nutritifs majeurs (N, P, K, S ...) ou mineurs (oligo-éléments) étant intimement liés au cycle du carbone (dégradation des celluloses, lignine, hémi-cellulose, pectine, protéine) et donc à la présence dans le sol de bactéries et champignons et leurs enzymes spécifiques, on comprend l'importance considérable de la matière organique fraîche et surtout de son évolution dans le sol.

La présente invention a pour objet un inoculum microbien contenant une forte concentration de microorganismes spécifiques et sélectionnés, à utiliser dans les sols notamment pour accélérer la dégradation des résidus de récolte enfouis, d'en améliorer la valeur humique et fertilisante, en augmentant l'assimilation de la fumure chimique.

Plus particulièrement, la présente invention concerne un inoculum microbien, utile notamment pour la dégradation des matières organiques dans les sols, composé d'un mélange de microorganismes de genres différents pouvant vivre dans le sol et non-pathogènes pour l'homme, les animaux et les végétaux, caractérisé en ce qu'il s'agit de microorganismes aérobies stricts ou aérobies facultatifs, et mésophiles, existant sous une forme résistante au stockage, dégradant la cellulose, l'hémi-cellulose, la lignine et les protéines composant les matières organiques d'origine végétale ou animale.

Les microorganismes selon la présente invention sont choisis de préférence dans les genres Cellvibrio, Bacillus, corynebacterium et Trichoderma.

D'après les cultures en laboratoire et les contrôles d'activité, en test de croissance et production végétale les souches suivantes constituent le mode de réalisation préféré de l'invention : Cellvibrio vulgaris (FF 12) et Trichoderma harzianum (FF 13) pour leur activité enzymatique cellulolytique élevée et Bacillus cereus (FF 7) et Corynebacterium Aquaticum (FF 5) pour leurs actions spécifiques observées lors des tests de minéralisation effectués en essais agricoles.

Le Trichoderma harzianum est utilisé selon la présente invention, de préférence sous forme de spores, alors que les bactéries sont de préférence immobilisées sur un support solide.

Mais, un problème majeur rencontré lors de la manipulation de microorganismes dans le domaine agricole est son mode de transport, de conservation, et d'utilisation.

Il est souvent fait appel à des supports solides, du type gel de polyacrylamide, qui sont fort coûteux, encombrants et non-naturels : Ces supports, outre leur coût élevé, ne se mêlent pas à la biomasse végétale. Or, le mélange microbien selon l'invention étant destiné à une utilisation agronomique, nécessite un support solide à base de produits naturels et peu coûteux.

Dans ce but, après de nombreux essais, on a découvert que le kaolin calciné, en particulier un kaolin dont la surface spécifique (évaluée par la méthode B.E.T.) est comprise entre, environ, 5 et $20m^2$/gramme, constituait un excellent support solide pour la fixation d'inoculum microbien ; ce kaolin a, de préférence, la granulométrie suivante (évaluée par la méthode BOUYOUOOS) : diamètre statistique médian de 1,5 à 8 microns ; il a aussi de préférence une perte au feu à 80°C de 0,2 à 0,8% ; ce kaolin calciné peut être, par exemple du type Costacal, obtenu aux Etablissements LAMBERT RIVIERE.

En conséquence, la présente invention concerne également un procédé de préparation d'une poudre stable de microorganismes, caractérisé en ce qu'on mélange une biomasse humide des microorganismes avec un kaolin calciné et en ce que l'on sèche le produit de mélange. Le mélange peut être effectué dans un malaxeur à vitesse lente ; la biomasse humide peut être obtenue par centrifugation après une concentration des bactéries dans leur milieu de culture ; cette biomasse comporte, de préférence 10 à 30% de matière sèche ; il est préférable aussi, d'ajouter le kaolin calciné à la biomasse humide pendant le mélange ; enfin, le produit du mélange peut être séché à une température permettant de maintenir la viabilité des bactéries, soit de préférence, à une température comprise entre 30°C et 40°C.

La matière sèche ainsi obtenue à partir des bactéries immobilisées sur kaolin calciné, comme décrit précédemment, est mélangée dans des proportions définies aux spores de Trichoderma afin d'obtenir l'inoculum selon l'invention.

L'inoculum ainsi obtenu comporte, de préférence, $10^9$ à $10^{11}$ bactéries par gramme de matière sèche et $10^8$ à $10^{10}$ spores de Trichoderma par gramme de matière sèche. Il peut être utilisé comme agent fertilisant, seul ou en association avec un support acceptable en agriculture, tel qu'un fertilisant minéral et/ou organique, ou une matière d'origine végétale et/ou animale, ou des résidus organiques végétaux et/ou des fumiers.

Les applications agricoles de cet agent fertilisant sont multiples et très générales, s'opposant ainsi à la trop grande spécificité des "biofertilisants" connus.

Cet agent fertilisant, en effet, accélère la dégradation des matières organiques végétales. A ce titre, il peut être épandu sur le sol que ce soit en plein champ ou sous serre, et son action cellulolytique agit sur des débris végétaux broyés ou non.

Il est intéressant d'enfouir des matières organiques et l'agent fertilisant, par un travail du sol en profondeur, ou même d'épandre directement l'agent fertilisant dans le sol. En effet, les gaz tels que l'ammoniac ($NH_3$) et le dioxyde de carbone ($CO_2$), produits par la décomposition organique, peuvent être ainsi "piégés" et réutilisés par les végétaux sous une forme minéralisée.

L'utilisation de l'agent fertilisant selon l'invention peut aussi être envisagée très avantageusement pour les cultures hydroponiques hors sol, sur tourbe ou paille. Dans ce cas, l'assimilation des éléments fertilisants est grandement améliorée.

L'agent fertilisant selon l'invention peut également être utilisé pour détoxifier les sols, en particulier lors de l'enfouissement de débris toxiques tels que les racines de sorgho.

Ainsi, le procédé de préparation de l'inoculum bactérien comprend les étapes suivantes :

    a) culture des microorganismes en fermenteur ;

    b) fixation sur support solide ;

    c) préparation de l'agent fertilisant.

a) <u>Culture des microorganismes en fermenteur</u>

    <u>Milieux de culture</u>

    1) Un milieu de culture optimum a été défini pour les 3 souches de bactéries considérées, sa composition est la suivante :

- Cerellose,                    40   g/l
- Soyamine,                     12   g/l
- $Na_2 HPO_4$,                 15   g/l
- $KH_2 PO_4$,                   6   g/l
- $SO_4 Mg 7H_2O$,               2   g/l
- Extrait de levure,           0,5g/l

2) L'étude des conditions de sporulation de la souche de Trichoderma harzianum a conduit au choix du milieu suivant :

- $(NH_4)_2 SO_4$,               5   g
- $KH_2 PO_4$,                   7   g
- $Mg SO_4 7 H_2O$,              1   g
- $CaCl_2, 2 H_2O$,              1   g
- Oligo-éléments MANDELS,       1   ml
- Extrait de levure à 5g/l,1    ml
- Pulpe de betterave
  + son,                        100g (50/50)
- Eau, Q.S.P. humidité,         65-70 %

Le pH de la solution des sels est ajusté à 3 avec de l'acide phosphorique.

Culture

Après stérilisation classique et ensemencement par un pied de cuve, la culture est poursuivie de 24 à 48 heures à une température de 18°C à 30°C avec une aération variant de 0,5 VVM à 1 VVM (Volume d'air par Volume utile par Minute).

b) Fixation sur support solide

Dès que la concentration maximum de bactérie est atteinte, la biomasse est récupérée par centrifugation et on obtient cette biomasse sous forme pâteuse à environ 20 % de matière sèche.

L'immobilisation sur support solide s'effectue par un mélange à parts égales biomasse humide et kaolin calciné (type Costacal obtenu aux Ets LAMBERT RIVIERE) avec les caractéristiques suivantes :

- perte au feu à 80°C : de 0,2 à 0,8 %,

- granulométrie (Méthode BOUYOUCOS) : Diamètre statistique médian de 1,5 à 8 microns.

Ce mélange est effectué dans un mélangeur genre malaxeur, à vitesse lente. Le kaolin calciné est ajouté lentement pour favoriser le mélange et permettre une meilleure homogénéité.

La poudre obtenue est alors séchée sous vide ou sous courant d'air sec à 30-40°C afin d'éliminer l'eau provenant de la biomasse humide.

On obtient ainsi un inoculum contenant de $10^{10}$ à $10^{12}$ bactéries par gramme de matière sèche.

Après contrôle de viabilité en tout de 7 mois le taux de viabilité est de 95 à 97 %.

Cette préparation est effectuée de façon identique pour les 3 souches de bactéries.

En ce qui concerne le Trichoderma harzianum, il est cultivé sur support solide dont les caractéristiques ont été définies précédemment. La culture se poursuit 7 à 8 jours afin d'obtenir la sporulation.

Après séchage à 35°C, on obtient une poudre contenant $8.10^{9}$ spores par gramme de produit sec.

Les 4 poudres sont alors mélangées intimement à parts égales afin d'obtenir l'inoculum microbien dont les caractéristiques sont les suivantes :

* Taux de bactéries : $5.10^{9}$ à $5.10^{10}$ bactéries par gramme de matière sèche

* Taux de spores : $10^{9}$ spores de Trichoderma harzianum.

## c) Préparation de l'agent fertilisant

Cet inoculum microbien peut alors être mélangé à des fertilisants minéraux ou organiques ou des organo-minéraux.

Le mélange ainsi obtenu pourra être granulé ou pelletisé (presse).

Le produit ainsi obtenu pourra être épandu sur le sol et enfoui avec les résidus de récolte (pailles, chaumes, cannes de maïs, de tournesol, de colza, de sorgho, résidus de betterave) ou tout autre résidu organique végétal provenant de l'industrie agro-alimentaire.

L'action des microorganismes ainsi épandus permet d'améliorer de façon notoire la dégradation de la matière organique présente dans ou sur le sol et ainsi d'en valoriser les qualités fertilisantes et humiques.

Des applications de cet agent fertilisant ainsi que les résultats obtenus sont donnés dans les exemples suivants.

## EXEMPLE 1

## Action de l'inoculum microbien

Les expérimentations de culture en pots de l'Agrostis ont été menées en laboratoire.

Des séries de répétitions ont permis d'observer les résultats suivants :

1) A fumure équivalente, l'introduction de 500 mg d'inoculum microbien par pot permet d'augmenter la production végétale de l'Agrostis de 43 % pour deux coupes consécutives.

2) La toxicité de broyat des racines de sorgho incorporé au support de culture a été éliminé par l'adjonction de 350 mg d'inoculum microbien par pot.

EXEMPLE 2

Action d'un mélange matière organique/inoculum microbien

   a) Fabrication de l'agent fertilisant

Le support organique a été préparé par compostage de pulpe de raisin et de fumier de bergerie puis séché à 95°C en four rotatif et enfin broyé.

A la poudre ainsi obtenue on a ajouté 1 % d'inoculum microbien.

Le mélange ainsi obtenu a été"pelletisé"à froid à la presse pour obtenir des "pellets" ou "bouchons" d'environ 3,5 mm de diamètre et 5 à 6 mm de long.

   b) Application

      A) En laboratoire

Des expérimentations ont été menées dans le cadre de l'étude du compostage aérobie strict des écorces de résineux.

Des mesures cinétiques ont montré que la dose optimum de l'agent fertilisant dont la préparation est décrite ci-dessus était de 2 à 5% de la quantité totale d'écorce (en matière sèche) et qu'il fallait y adjoindre de 0,5 à 0,75 % d'urée.

Dans ces conditions, la durée de compostage est diminuée de moitié.

      B) En plein champ et sous serre

On a utilisé le même agent fertilisant que précédemment.

      1) Culture sous serre

Ces applications sous serre froide ont été conduites sur culture de laitue.

En préparation de sol, 2 000 kg à l'hectare d'engrais organo-minéral 3.10.10 et 400 kg de Patenkali

0223661

ont été utilisés. Sur les parcelles traitées l'agent fertilisant a été amené à raison de 500 kg/ha.

En 60 jours de culture, le gain moyen par salade a été de 125 g (345 g contre 220 g sur les témoins).

Des résultats analogues ont été obtenus avec la culture des fraises, des tomates et des endives.

2) Culture_en_plein_champ

a) Dégradation_de_la_matière_organique

L'agent fertilisant précédemment décrit est utilisé à raison de 400 kg/ha lors du déchaumage après broyage des pailles, chaumes ou cannes de maïs ou colza et céréales.

Après épandage de l'agent fertilisant le sol est travaillé sur une profondeur de 15 à 25 cm afin d'enfouir matière organique et agent fertilisant.

Les observations effectuées ont montré une nette accélération de la dégradation des pailles, chaumes et cannes, même sur terre battante.

b) Amélioration_de_la_productivité

- Blé sur Maïs : variété FESTIVAL        témoin : 91 quintaux traité :107 quintaux

Gain : 16 quintaux

- Blé sur blé : variété HARDY        témoin : 77,2 quintaux traité : 84,6 quintaux

Gain : 7,4 quintaux

- Vigne : les applications de l'agent fertilisant ont été effectuées à raison de 600 kg/ha avec des écorces de résineux semi-compostées, et ce à l'automne.

Les résultats ont montré un gain moyen de 1 200 kg de raisins à l'hectare. Il est à noter que l'on a observé un fort effet antichlorotique.

EXEMPLE 3 : Composition et analyse microbiologique d'un agent fertilisant selon l'invention

1) On prépare un agent fertilisant ayant la composition suivante :

SUPPORT (pourcentages exprimés par rapport à la totalité de l'agent fertilisant)
. Pulpe de fruits préfermentée dont
       - matière organique totale ....... 49,5 %
       - azote total ................... 3,0 %
         dont azote organique .......... 2,2 %
. Oligo-élements ......................... 0,4 %
. Acides aminés .......................... 1,2 %

INOCULUM MICROBIEN : 1 à 2 milliards de microorganismes par gramme d'agent fertilisant, soit 0,5 % à 1 % d'inoculum par rapport à la totalité d'agent fertilisant, dont :
. Flavobactérium ..................... 0,4 à 0,6.$10^9$ bact/gr
. Bacillus ........................... 0,4 à 0,6.$10^9$ bact/gr
. Cellvibrio ......................... 0,2 à 0,3.$10^9$ bact/gr
. Trichoderma ........................ $10^4$ spores/gr.

Le mélange ainsi obtenu est présenté sous forme de pellets de 3,5 mm de diamètre.
2) De l'analyse microbiologique de cet agent fertilisant effectuée par la méthode de RUSCH (méthodes de culture biologique), il ressort les caractérisques suivantes :
VALEUR NUTRITIVE

La valeur selon le test était de 2800. Dans les tableaux cela correspond à très bien. Ce chiffre donne le nombre de bactéries qui se développent dans de l'eau ordinaire, selon le procédé standard. Plus il y a de bactéries, plus la valeur nutritive sera grande quand celles-ci meurent.

Les meilleurs composts apparus sur le marché jusqu'à maintenant avaient seulement une valeur de 2000.

FERTILITE

La valeur selon le test était de 8900. Dans les tableaux cela correspond à très bien. Ce chiffre donne le nombre de bactéries qui se développent dans de l'eau sucrée selon le procédé standard. Plus il y a de bactéries, plus il y en aura qui vivent en contact étroit avec les racines. Ici encore la valeur est plus élevée que celle des meilleurs composts qui n'ont qu'une valeur de 5000.

Selon les tableaux, il est possible d'appliquer une méthode de culture plus biologique, c'est-à-dire avec moins d'engrais chimiques et moins de pesticides.

EXEMPLE 4 : Influence de l'agent fertilisant de l'exemple 3 sur le compostage

1. Préparation des tests

Afin de savoir dans quelle mesure l'agent fertilisant de l'exemple 3 influence la décomposition de la matière organique, on a préparé dans le laboratoire un test de compostage.

Comme matière organique, on a utilisé de l'écorce d'arbre, celle-ci étant connue comme la matière organique la plus dure. Cette écorce a été mise dans deux simulateurs de compostage et on a suivi la décomposition pendant 10 jours à 25°C.

Dans l'un des deux simulateurs, on a ajouté 5 % de l'agent fertilisant de l'exemple 3.

Pour connaître le degré de décomposition, on a mesuré la quantité d'oxygène utilisée pendant celle-ci. Chaque 3 h 30 on a fait des mesures afin de suivre le processus.

2. Résultat des tests

On peut constater une différence entre la vitesse de compostage avec et sans l'agent fertilisant de l'exemple 3.

A - Les deux premiers jours

Pendant cette période, la matière la plus facile à décomposer a été décomposée, ce sont les sucres et les albumines présents dans l'écorce.

La vitesse de décomposition est environ 30 % plus élevée avec l'agent étudié que sans lui. Ceci prouve que la matière organique qui se décompose facilement, se décomposera 30 % plus vite avec l'agent fertilisant selon la présente invention.

B- Après le deuxième jour

A partir de ce moment commence la décomposition du matériel qui est plus difficile à décomposer, comme la cellulose et la lignine. Celles-ci sont les matières les plus importantes pour la formation de l'humus. La vitesse de décomposition sans l'agent selon la présente invention diminue jusqu'à moins de 50 % par rapport à la vitesse des deux premiers jours.

L'écorce traitée avec l'agent de l'exemple 3 continue à avoir la même vitesse de décomposition que celle des deux premiers jours. Ainsi, après 10 jours, la décomposition a été le double par rapport à celle du contrôle. Finalement la vitesse de décomposition de la matière dure de l'écorce est environ 3 fois plus grande avec l'agent étudié que sans. L'agent de l'exemple 3 contient donc des bactéries plus aptes à décomposer la matière dure que les bactéries qui le font normalement.

Puisque c'est la matière la plus dure qui fournit la plus grande quantité d'humus, l'agent fertilisant selon l'invention est très apte à produire plus rapidement de l'humus.

EXEMPLE 5 : Action de l'agent fertilisant de l'exemple 3

. Test sur tomates en serres

1) INFORMATIONS TECHNIQUES

Désinfection du sol en 1984 avec bromure de méthyl.

Engrais organique : 1000 kg de fumier par are

Analyse du sol : pH : 6,2        Ca : 112    Mg 21,5 p : 71          Sel : 150 K : 47          % C : 2,16

Engrais chimiques : 3 kg $K_2SO_4$, 2 kg Floranid et

2 kg $KN\ O_3$ par are

Variété : Vedettos

Plantées le : 23 mai 1985

Plantées à une densité de : 2 plants par $m^2$

Agents fertilisants de l'exemple 3 : 400 kg/ha à une profondeur de 15 cm, le 22 mai 1985.

2) OBSERVATIONS DES PLANTES

On n'a pas constaté de différences significatives entre les
plantes, ou de maladies pendant la période de croissance.
On a cependant constaté des différences significatives de
récolte.

3) MESURES

Le test a été exécuté à 3 reprises sur 10 $m^2$ chacun. Ceci
signifie 20 plantes à chaque fois. Les lignes du bord n'ont
pas été récoltées de sorte que les chiffres sont dérivés
de 3 x 10 = 30 plantes.

La première récolte a eu lieu le 23.07.1985, la dernière
le 7.10.1985. Au total, il y a eu 24 récoltes. A chaque
date de récolte on a noté les poids et le nombre de
fruits par classe de qualité.

Les classes de qualité répondent aux normes suivantes :

| CLASSE | TAILLE DU FRUIT (mm) | QUALITE |
|---|---|---|
| EE | 70 - 80 | Extra |
| A | 60 - 70 | 1ère ou 2ème classe |
| B | 50 - 60 | 1ère ou 2ème classe |
| C | 45 - 50 | 1ère ou 2ème classe |
| "potage" | moins de 45 | 1ère ou 2ème classe |
| "Gros morceaux" | plus de 80 | 3ème classe |

## 4. DONNEES SUR LE RAPPORT

### A) Rapport en poids

| TRAITEMENT | RECOLTE EN kg | RAPPORT EN % |
|---|---|---|
| Témoin | 106,70 | 100 |
| Agent fertilisant de l'exemple 3 | 121,95 | 114 |

### B) Divisé par classe de qualité

| Classe | EE | A | B | C | "Potage" | "Gros morceaux" |
|---|---|---|---|---|---|---|
| Témoin | 69 | 265 | 328 | 221 | 164 | 53 |
| Agent fertilisant de l'exemple 3 | 110 | 342 | 147 | 77 | 110 | 58 |

### C) Taille des fruits

| Traitement | Récolte en kg | Nombre de fruits | Poids moyen des fruits |
|---|---|---|---|
| Contrôle | 106,70 | 1100 | 97 g |
| Agent fertilisant de l'exemple 3 | 121,95 | 844 | 114 g |

0223661

- 17 -

5. INTERPRETATION DES RESULTATS

L'agent selon l'invention a une influence positive sur le rapport en poids, ainsi que sur la qualité des tomates.

Le rapport en poids est de 14 % plus élevé ; il y avait donc une meilleure nourriture pour les plantes.

Le nombre de fruits a diminué, ce qui signifie qu'on était déjà près du maximum pour les tomates. La plante règle naturellement le nombre de ses fruits. Dans le cas d'un trop grand nombre de fruits, la plante rejette les petits, afin de pouvoir nourrir optimalement les grands On voit donc un glissement vers une classe de qualité supérieure.

Ce glissement se passe également pour la répartition par taille de fruits où l'agent fertilisant étudié jou un rôle intéressant. En effet, le nombre de tomates dans les catégories EE et A, les plus intéressantes, est nettement plus grand avec ledit agent, alors que dans les classes B, C et "Potage" c'est l'inverse.

L'agent fertilisant a un bon effet en serres où l'on fait régulièrement une désinfection du sol. Un rétablissement de la vie du sol peut considérablement améliorer la nutrition des plantes. Un déblocage du processus de décomposition augmente la disponibilité des éléments nutritifs qui ne le sont même pas assez avec un apport d'engrais chimiques.

. Test sur betteraves sucrières

1. INFORMATIONS TECHNIQUES

Culture précédente : Orge d'hiver

Fumure verte : 30 tonnes de fumier d'étable

Analyse du sol : $P_2O_5$ = 28     CaO = 785

$K_2O$ = 16     Ph-KCl = 7,8 MgO = 9     Humus (%C) = 1,8

Date de labour : fin mars

Azote : 1ère dose : 70 u    2ème dose : 55 u

Semées le : 22.04.1985

Semées à distance de : 17 cm x 45 cm

Variété : Allyx Agent de l'exemple 3 : 150 kg/ha avant la préparation du sol

## 2. MESURES

Le test a été exécuté à 3 reprises sur 600 m$^2$ chacun. A chaque reprise on a pris 15 bandes de 10 m de longueur. On a noté les poids et déterminé la teneur en sucre. Ces chiffres ont été extrapolés par rapport aux récoltes par Ha, pour les racines ainsi que pour les sucres.

| | *Contrôle* | | | Agent fertilisant de l'exemple 3 | | |
|---|---|---|---|---|---|---|
| | *Rapports Racines kg/ha* | *Teneur Sucre %* | *Rapport Sucre kg/ha* | *R. R. kg/ha* | *T. S. %* | *R. S. kg/ha* |
| *Parcelle 1 :* | 56,881 | 17,26 | 9.818 | 59.629 | 18,32 | 10.924 |
| *Parcelle 2 :* | 59,391 | 17,73 | 10.530 | 61.341 | 18,16 | 11.140 |
| *Parcelle 3 :* | 57.513 | 17,85 | 10.266 | 59.328 | 17,61 | 10.448 |
| *Moyenne :* | 57.928 | 17,61 | 10.205 | 60.099 | 18,03 | 10.838 |
| *Différence :* | | | | + 2.171 kg | + 0,42 % | + 633 kg |

## 3. COMMENTAIRES SUR LES RESULTATS

Le rapport en racines ainsi que le rapport en sucre est plus élevé après application d'agent fertilisant selon la présente invention.

AUTRES TESTS

De nombreux essais, similaires à ceux précédemment décrits, ont été effectués.

On a ainsi constaté les actions favorables de l'agent fertilisant de l'exemple 3 sur la germination et l'implantation du maïs ainsi que sur la minéralisation de l'azote.

DEPOT DE SOUCHES

Les souches suivantes ont été déposées le 11 octobre 1985 à la Collection Nationale de Cultures de Microorganismes de l'Institut Pasteur, 28, rue du Docteur-Roux, 75724 PARIS CEDEX 15, sous les numéros suivants :

- pour la souche FF 5, Corynebacterium Aquaticum (précédemment identifiée comme étant probablement xanthomonas sp )..................... n° I-480
- pour la souche FF 7, Bacillus Cereus variété Mycoides .................... n° I-481
- pour la souche FF 12, pseudomonas sp (cellvibrio vulgaris) ............... n° I-482
- pour la souche FF 13, Trichoderma harzianum........................... n° I-495

## REVENDICATIONS

1) Inoculum microbien utile notamment pour la dégradation des matières organiques dans les sols, composé d'un mélange de microorganismes de genres différents pouvant vivre dans le sol et non-pathogènes pour l'homme, les animaux et les végétaux, caractérisé en ce qu'il s'agit de microorganismes aérobies stricts ou aérobies facultatifs, et mésophiles, existant sous une forme résistante au stockage, dégradant la cellulose, l'hémi-cellulose, la lignine et les protéines composant les matières organiques d'origine végétale ou animale.

2) Inoculum selon la revendication 1, caractérisé en ce que les microorganismes sont choisis dans les genres Cellvibrio, Bacillus, Corynebacterium et Trichoderma.

3) Inoculum selon la revendication 2, caractérisé en ce qu'il est constitué d'un mélange de Cellvibrio vulgaris, Bacillus cereus et Corynebacterium Aquaticum et Trichoderma harzianum.

4) Inoculum selon la revendication 3, caractérisé en ce que Trichoderma harzianum est sous forme de spores.

5) Inoculum selon la revendication 3, caractérisé en ce que les bactéries sont immobilisées sur un support solide.

6) Inoculum selon la revendication 5, caractérisé en ce que le support solide est du kaolin calciné.

7) Inoculum selon la revendication 6, caractérisé en ce que le kaolin calciné a une surface spécifique comprise entre 5 et 20 $m^2$/gramme.

8. Inoculum selon l'une des revendications 3 à 7, caractérisé en ce qu'il contient de $10^9$ à $10^{11}$ bactéries par gramme de matière sèche et $10^8$ à $10^{10}$ spores de Trichoderma par gramme de matière sèche.

9. Agent fertilisant caractérisé en ce qu'il est constitué d'un inoculum selon l'une des revendications 1 à 8, seul ou en mélange avec un support acceptable en agriculture.

10. Agent fertilisant selon la revendication 9, caractérisé en ce que le support acceptable en agriculture comporte des fertilisants minéraux et/ou organiques.

11. Agent fertilisant selon l'une des revendications 9 et 10, caractérisé en ce que le support comporte des matières d'origine animale et/ou végétale.

12. Agent fertilisant selon la revendication 11, caractérisé en ce que le support comporte des résidus organiques végétaux et/ou des fumiers.

13. Agent fertilisant selon l'une des revendications 9 à 12, caractérisé en ce que l'inoculum représente de 0,1 à 5 % en poids de l'agent fertilisant.

14. Procédé de préparation d'une poudre stable de bactéries entrant dans la constitution de l'inoculum selon les revendications 6 ou 7, caractérisé en ce qu'on mélange une biomasse humide des microorganismes avec un kaolin calciné et en ce que l'on sèche le produit du mélange.

15. Procédé selon la revendication 14, caractérisé en ce que le mélange est effectué dans un malaxeur à vitesse lente.

16. Procédé selon l'une des revendications 14 et 15, caractérisé en ce que la biomasse humide comporte de 10 à 30 % de matière sèche.

17. Procédé selon l'une des revendications 14 à 16, caractérisé en ce que le kaolin calciné est ajouté pendant le mélange à la biomasse humide.

18. Procédé selon l'une des revendications 14 à 17, caractérisé en ce que le produit du mélange est séché sous vide à une température comprise entre 30 et 40°C.

19. Procédé pour l'amélioration des sols, caractérisé en ce qu'il consiste à épandre sur ou dans les sols, ou à enfouir avec des matières organiques végétales, un agent fertilisant selon l'une des revendications 9 à 13.

20. Procédé pour l'amélioration des sols, selon la revendication 19, caractérisé en ce que les matières organiques végétales sont des résidus de récolte.

21. Procédé pour l'amélioration de l'assimilation des éléments fertilisants, caractérisé en ce qu'il consiste à utiliser un agent fertilisant selon l'une des revendications 9 à 13, en culture hydroponique hors sols sur tourbe ou paille.

22. Application d'un agent fertilisant selon l'une des revendications 9 à 13 à l'accélération du compostage des déchets végétaux.

0223661

Office européen
des brevets

**RAPPORT DE RECHERCHE EUROPEENNE**

Numero de la demande

EP  86 40 2304

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 125 073 (NATIONAL RESEARCH DEVELOPMENT CORPORATION)<br><br>* Revendications 1,3; page 11, lignes 25-28; page 10, ligne 34 - page 11, ligne 2 * | 1,2,4, 9,10, 19,20, 22 | C 05 F 11/08<br>C 12 N 1/04 |
| Y | | 14-18 | |
| Y | FR-A-2 096 887 (H. GRIFFON)<br>* Revendications 1-3 * | 14-18 | |
| Y | FR-A-2 559 478 (J.O. HARROP)<br>* Revendications 1,5,7 * | 14-18 | DOMAINES TECHNIQUES RECHERCHES (Int. Cl.4) |
| X | CHEMICAL ABSTRACTS, vol. 105, no. 4, 28 juillet 1986, page 326, résumé no. 29244e, Columbus, Ohio, US; Y. OHTA et al.: "Rapid microbial deodorization of agricultural and animal wastes", & EUR. CONGR. BIOTECHNOL., 3rd 1984, 3, 129-34<br>* Résumé en entier * | 1,2 | C 05 F 9/00<br>C 05 F 11/00<br>C 12 N 1/00<br>C 12 R 1/00 |

Le présent rapport de recherche a été établi pour toutes les revendications

| Lieu de la recherche | Date d'achèvement de la recherche | Examinateur |
|---|---|---|
| LA HAYE | 21-01-1987 | BOUTRUCHE J.P.E. |